# EUROPEAN PATENT SPECIFICATION

(11) **EP 3 701 998 B1**
(45) Date of publication and mention of the grant of the patent: **03.03.2021**
(21) Application number: 20159209.4
(22) Date of filing: 25.02.2020
(51) Int. Cl.: A61M 37/00

(54) **MANUFACTURING METHOD OF MICRONEEDLE ARRAY**
HERSTELLUNGSVERFAHREN EINER MIKRONADELANORDNUNG
PROCÉDÉ DE FABRICATION DE RÉSEAU DE MICRO-AIGUILLES

(30) Priority: 28.02.2019 JP 2019036376
(43) Date of publication of application: 02.09.2020
(73) Proprietor: FUJIFILM Corporation, Tokyo 106-8620 (JP)
(72) Inventor: TAKANO, Ikuo, Kanagawa, 250-0001 (JP)
(74) Representative: Klunker IP Patentanwälte PartG mbB

(56) References cited:
- EP-A1- 3 235 537
- EP-A1- 3 270 121
- WO-A2-2007/030477
- AU-B2- 2014 200 648
- US-A1- 2007 086 021

## Description

### BACKGROUND OF THE INVENTION

### 1. Field of the Invention

The present invention relates to a manufacturing method of a microneedle array, and more particularly to a manufacturing method of a microneedle array in which a drug solution is ejected from an ejection nozzle into a recessed portion of a mold.

### 2. Description of the Related Art

In recent years, a microneedle array (percutaneous absorption sheet) formed with needle-like protruding portions (also referred to as small needles or microneedles) containing a drug has been used to deliver the drug into the skin. In general, by pressing the microneedle array against the skin to insert the needle-like protruding portions into the skin, the drug of the needle-like protruding portions is delivered into the skin.

As a method of manufacturing the microneedle array, a mold in which needle-like recessed portions having an inverted shape of the needle-like protruding portions is formed. A method of forming a microneedle array by filling needle-like recessed portions of a mold with a solution containing a drug (also referred to as a drug solution), drying the solution, applying a solution not containing the drug (also referred to as a base material solution), and drying the solution is known.

In the method of manufacturing the microneedle array, since the filling amount of the drug solution is associated with the drug dose, it is necessary to reliably fill the needle-like recessed portions of the mold with a very small amount of the drug solution in a constant amount with high accuracy.

Hitherto, a method for filling a drug solution has been proposed (refer to JP2016-112169A).

### SUMMARY OF THE INVENTION

JP2016-112169A discloses a filling method in which liquid droplets are ejected from an ejection nozzle into needle-like recessed portions of a mold. However, when the filling is performed at a high velocity in order to increase the productivity, there is a problem that the landing position of the liquid droplet and the position of the needle-like recessed portion deviate from each other.

Such a positional deviation is caused by a landing positional deviation due to apparatus vibration that occurs when a stage or an ejection nozzle is moved at a high velocity, a landing positional deviation due to a change in liquid droplet flight velocity caused by a state change in the ejection nozzle over time and a change in a drug solution over time, a positioning deviation during mold installation, a positional deviation of needle-like recessed portions due to expansion and contraction or strain of the mold itself, and combinations thereof. It is practically difficult to eliminate these factors and completely prevent the positional deviation.

In a case where the drug solution deviates from the center portion of the needle-like recessed portion or the drug solution adheres to the wall surface of the needle-like recessed portion due to the positional deviation described above and thus the drug solution cannot close the needle-like recessed portion, the drug solution cannot fill the distal end of the needle-like recessed portion, and there is a problem that the manufacturing yield (yield) of a microneedle array is reduced.

The present invention has been made taking the above circumstances into consideration, and an object thereof is to provide a manufacturing method of a microneedle array in which the distal end of a needle-like recessed portion can be filled with a drug solution even in a case where a positional deviation between a landing position of a liquid droplet and the needle-like recessed portion occurs.

A manufacturing method of a microneedle array according to a first aspect comprises: an adjustment step of positioning and adjusting a mold having a first surface and a second surface, which oppose each other, and a plurality of needle-like recessed portions in the first surface, and an ejection nozzle which ejects a drug solution in a first direction; a relative movement step of moving the mold and the ejection nozzle relative to each other to cause a position of the needle-like recessed portion and a position of the ejection nozzle to coincide with each other in a plan view in the first direction; an ejection step of ejecting the drug solution from the ejection nozzle toward the needle-like recessed portion; a vibration step of vibrating the mold to move the drug solution toward the needle-like recessed portion and close the needle-like recessed portion with the drug solution after the ejection step; and a suction step of suctioning the second surface of the mold.

According to the first aspect, the drug solution closes the needle-like recessed portion, and fills the needle-like recessed portion by suction. The manufacturing yield of the microneedle array is improved.

In the manufacturing method of a microneedle array according to a second aspect, the vibration in the vibration step is a horizontal reciprocating motion. In the second aspect, the vibration of the horizontal reciprocating motion can be applied to the mold.

In the manufacturing method of a microneedle array according to a third aspect, a movement direction of the drug solution and a direction of the horizontal reciprocating motion are the same direction. In the third aspect, in the horizontal reciprocating motion, the drug solution can be moved to the needle-like recessed portion and close the needle-like recessed portion more reliably within a short period of time.

In the manufacturing method of a microneedle array according to a fourth aspect, the vibration in the vibration step is a horizontal circular motion. In the fourth aspect, the vibration of the horizontal circular motion can be applied to the mold.

In the manufacturing method of a microneedle array according to a fifth aspect, the vibration in the vibration step is a vertical reciprocating motion. In the fifth aspect, the vibration of the vertical reciprocating motion can be applied to the mold.

In the manufacturing method of a microneedle array according to a sixth aspect, the vibration in the vibration step further includes a vertical reciprocating motion. In the sixth aspect, the vibration of a combination of the horizontal reciprocating motion and the vertical reciprocating motion can be applied to the mold.

In the manufacturing method of a microneedle array according to a seventh aspect, the vibration in the vibration step further includes a vertical reciprocating motion. In the seventh aspect, the vibration of a combination of the horizontal circular motion and the vertical reciprocating motion can be applied to the mold.

In the manufacturing method of a microneedle array according to an eighth aspect, the needle-like recessed portion comprises: a cup portion provided in the first surface of the mold; and a distal end recessed portion which is connected to the cup portion and has a tapered shape in a depth direction of the mold, an edge portion of an opening of the needle-like recessed portion is chamfered, and a chamfer of the edge portion has a radius of curvature of 30 µm or more and 300 µm or less. In the eighth aspect, the drug solution that has landed on the edge portion can be collected in the needle-like recessed portion.

In the manufacturing method of a microneedle array according to a ninth aspect, the vibration in the vibration step has an amplitude of 1 mm or more and 10 mm or less, a frequency of 15 Hz or higher and 100 Hz or lower, and a time of 1 second or longer and 180 seconds or shorter. In the ninth aspect, by setting the amplitude, frequency, and time of the vibration to the above-described ranges, the drug solution can be moved to the needle-like recessed portion and close the needle-like recessed portion more reliably.

The manufacturing method of a microneedle array according to a tenth aspect, further comprises: after the suction step, a drug solution drying step of drying the drug solution filling the needle-like recessed portion; after the drug solution drying step, a base material solution filling step of filling the mold with a base material solution; and a base material solution drying step of drying the filled base material solution. In the tenth aspect, the microneedle array containing the drug solution can be manufactured.

According to the present invention, since the drug solution can be moved to the needle-like recessed portion and close the needle-like recessed portion, the distal end of the needle-like recessed portion of the mold can be filled with the drug solution, thereby improving the manufacturing yield of the microneedle array.

### BRIEF DESCRIPTION OF THE DRAWINGS

Fig. 1 is a perspective view illustrating a schematic configuration of a microneedle array.
Fig. 2 is an enlarged partial cross-sectional view of the microneedle array.
Fig. 3 is a perspective view illustrating an example of a mold.
Fig. 4 is a cross-sectional view taken along line IV-IV in Fig. 3.
Fig. 5 is a flowchart showing each step of a manufacturing method of a microneedle array.
Fig. 6 is a schematic configuration diagram of a drug solution filling apparatus used in a drug solution filling step.
Fig. 7 is a block diagram illustrating an electrical configuration of the drug solution filling apparatus.
Fig. 8 is a flowchart showing each step included in the drug solution filling step.
Fig. 9 is a schematic view illustrating a part of the steps included in the drug solution filling step.
Fig. 10 is a schematic view illustrating a part of the steps included in a drug solution filling step.
Fig. 11 is a schematic view illustrating a part of the steps included in the drug solution filling step.
Fig. 12 is a schematic view illustrating a part of the steps included in the drug solution filling step.
Fig. 13 is a schematic view illustrating a vibration step included in the drug solution filling step.
Fig. 14 is a schematic view illustrating the vibration step included in the drug solution filling step.
Fig. 15 is a schematic view illustrating the vibration step included in the drug solution filling step.
Fig. 16 is a schematic view illustrating the vibration step included in the drug solution filling step.
Fig. 17 is a schematic view illustrating the vibration step included in the drug solution filling step.
Fig. 18 is a schematic view illustrating the vibration step included in the drug solution filling step.
Fig. 19 is a schematic view illustrating the vibration step included in the drug solution filling step.
Fig. 20 is a waveform diagram showing an example of vibration.
Fig. 21 is a waveform diagram showing another example of the vibration.
Fig. 22 is a waveform diagram showing another example of the vibration.
Fig. 23 is a cross-sectional view for describing the shape of a needle-like recessed portion according to an example.
Fig. 24 is a table showing parameters at each level and evaluation results thereof.

### DESCRIPTION OF THE PREFERRED EMBODIMENTS

Hereinafter, preferred embodiments of the present invention will be described with reference to the accompanying drawings. The present invention is described by the following preferred embodiments. Modifications can be made by various methods without departing from the scope of the present invention, and other embodiments than the embodiments can also be used. Therefore, all modifications within the scope of the present invention are included in the appended claims.

Here, in the figures, like elements having similar functions are denoted by like reference numerals. In addition, in this specification, in a case where a numerical value range is expressed using "to", the numerical value range includes the numerical values of the upper limit and the lower limit indicated by "to".

### Microneedle Array

An example of a microneedle array (percutaneous absorption sheet) manufactured by a manufacturing method in the present embodiment will be described.

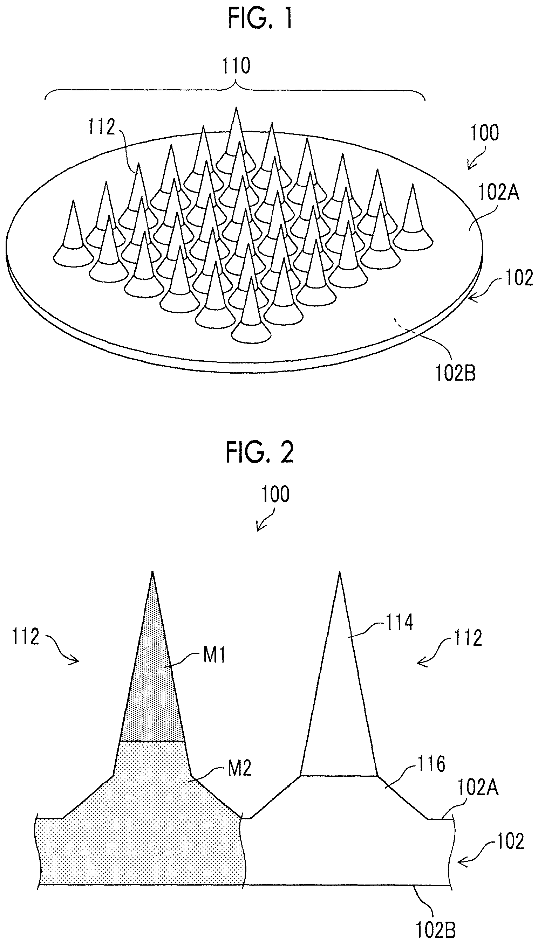

Fig. 1 is a perspective view illustrating an example of a microneedle array 100. The microneedle array 100 corresponds to a patch for one administration. The microneedle array 100 comprises a sheet portion 102 having a first surface 102A and a second surface 102B which oppose each other, and needle-like protruding portions 112 arranged on the first surface 102A of the sheet portion 102.

The sheet portion 102 has a thin flat shape as a whole with respect to the two opposing first and second surfaces 102A and 102B having a large area. Although the sheet portion 102 illustrated in Fig. 1 is circular in a plan view, the sheet portion 102 may be rectangular, polygonal, elliptical, or the like. Here, the plan view means a state in which the first surface 102A is observed in a direction orthogonal to the first surface 102A.

A plurality of the needle-like protruding portions 112 are arranged on the first surface 102A of the sheet portion 102 in a predetermined pattern. The plurality of arranged needle-like protruding portions 112 constitute an array pattern 110. For example, the array pattern 110 can be configured by arranging the plurality of needle-like protruding portions 112 in a concentric shape, and can also be configured by arranging the plurality of needle-like protruding portions 112 in a lattice shape. The array pattern 110 is not particularly limited and can be changed as appropriate.

The array pattern 110 is constituted by, for example, 4 to 2500 needle-like protruding portions 112. However, the number of needle-like protruding portions 112 is not limited to this number.

The needle-like protruding portion 112 is configured in a shape having a narrow distal end as compared with the root in contact with the sheet portion 102. Examples of the shape of the needle-like protruding portion 112 include a cone shape, a polygonal pyramid shape (such as a quadrangular pyramid shape), or a spindle shape. The overall shape of the needle-like protruding portion 112 may be a cone shape or a polygonal pyramid shape (such as a quadrangular pyramid shape), or may be a structure in which the inclination (angle) of the side surface of the needle portion is continuously changed. Alternatively, a multilayer structure of two or more layers in which the inclination (angle) of the side surface of the needle portion changes discontinuously may also be adopted.

Fig. 2 is an enlarged partial cross-sectional view of the microneedle array 100. The needle-like protruding portion 112 includes a needle portion 114 on the distal end side and a frustum portion 116 on the root side. The needle-like protruding portion 112 has a so-called two-stage structure in which the inclination of the side surface of the needle portion 114 and the inclination of the side surface of the frustum portion 116 discontinuously change in appearance.

The frustum portion 116 has two bottom surfaces and has a three-dimensional structure surrounded by a conical surface. The bottom surface (lower bottom surface) of the two bottom surfaces of the frustum portion 116 having a large area is connected to the sheet portion 102. The bottom surface (upper bottom surface) of the two bottom surfaces of the frustum portion 116 having a small area is connected to the needle portion 114. That is, of the two bottom surfaces of the frustum portion 116, the area of the bottom surface in a direction away from the sheet portion 102 is small.

The needle portion 114 has a bottom surface with a large area and a shape having a narrowest area at the distal end apart from the bottom surface. Since the bottom surface of the needle portion 114 having a large area is connected to the upper bottom surface of the frustum portion 116, the needle portion 114 has a tapered shape in a direction away from the frustum portion 116.

In the form of Figs. 1 and 2, the needle portion 114 has a cone shape, and the frustum portion 116 has a truncated cone shape. However, the needle portion 114 and the frustum portion 116 are not limited to these shapes. Depending on the degree of insertion of the needle portion 114 into the skin, the shape of the distal end of the needle portion 114 can be appropriately changed to a curved surface, a flat surface, or the like.

For example, a columnar pedestal portion can be provided between the frustum portion 116 and the sheet portion 102. In addition, a columnar intermediate portion can be provided between the needle portion 114 and the frustum portion 116.

The height (length) of the needle-like protruding portion 112 is represented by the length of a segment from the distal end of the needle-like protruding portion 112 perpendicular to the sheet portion 102. The height (length) of the needle-like protruding portion 112 is not particularly limited, but is preferably 350 µm or more and 2500 µm or less.

The microneedle array 100 is made of a first material M1 containing a drug and a second material M2 not containing the drug. The needle portion 114 has a distal end portion made of the first material M1 and a root portion made of the second material M2. The frustum portion 116 and the sheet portion 102 are made of the second material M2.

Containing a drug means containing a drug in an amount that exhibits the drug effect in a case where the body surface is punctured. In addition, not containing a drug means not containing a drug in an amount that exhibits the drug effect. In a case of not containing a drug, the range of the amount of the drug is a range from 0, at which the drug is not contained at all, to the amount at which the drug effect is not exhibited.

The drug is not limited as long as the drug has a function as a drug. In particular, it is preferable to select from pharmaceutical compounds belonging to peptides, proteins, nucleic acids, polysaccharides, vaccines, and water-soluble low-molecular compounds.

The first material M1 is not particularly limited, and examples thereof include polysaccharides, polyvinyl pyrrolidone, polyoxyethylene polyoxypropylene glycol, polyethylene glycol, polyvinyl alcohol, and proteins (for example, gelatin). Examples of the polysaccharides include hyaluronic acid, sodium hyaluronate, pullulan, dextran, dextrin, chondroitin sulfate, sodium chondroitin sulfate, cellulose derivatives (for example, water-soluble cellulose derivatives partially modified from cellulose, such as carboxymethyl cellulose, hydroxypropyl cellulose, and hydroxypropyl methylcellulose), hydroxyethyl starch, and gum arabic. The above components may be used singly or as a mixture of two or more.

Among the above components, the first material M1 is preferably at least one selected from the group consisting of hydroxyethyl starch, dextran, chondroitin sulfate, sodium chondroitin sulfate, sodium hyaluronate, carboxymethyl cellulose, polyvinyl pyrrolidone, polyoxyethylene polyoxypropylene glycol, polyethylene glycol, and polyvinyl alcohol, and is particularly preferably sodium chondroitin sulfate.

The second material M2 is not particularly limited, and examples thereof include polysaccharides, polyvinyl pyrrolidone, polyoxyethylene polyoxypropylene glycol, polyethylene glycol, polyvinyl alcohol, and proteins (for example, gelatin). Examples of the polysaccharides include hyaluronic acid, sodium hyaluronate, pullulan, dextran, dextrin, chondroitin sulfate, sodium chondroitin sulfate, cellulose derivatives (for example, water-soluble cellulose derivatives partially modified from cellulose, such as carboxymethyl cellulose, hydroxypropyl cellulose, and hydroxypropyl methylcellulose), hydroxyethyl starch, and gum arabic. The above components may be used singly or as a mixture of two or more.

Among the above components, the second material M2 is preferably at least one selected from the group consisting of hydroxyethyl starch, dextran, chondroitin sulfate, sodium chondroitin sulfate, sodium hyaluronate, carboxymethyl cellulose, polyvinyl pyrrolidone, polyoxyethylene polyoxypropylene glycol, polyethylene glycol, and polyvinyl alcohol, and is particularly preferably sodium chondroitin sulfate.

The first material M1 and the second material M2 may be the same material or different from each other.

### Manufacturing Method of Microneedle Array

Next, a manufacturing method of the microneedle array will be described with reference to the drawings.

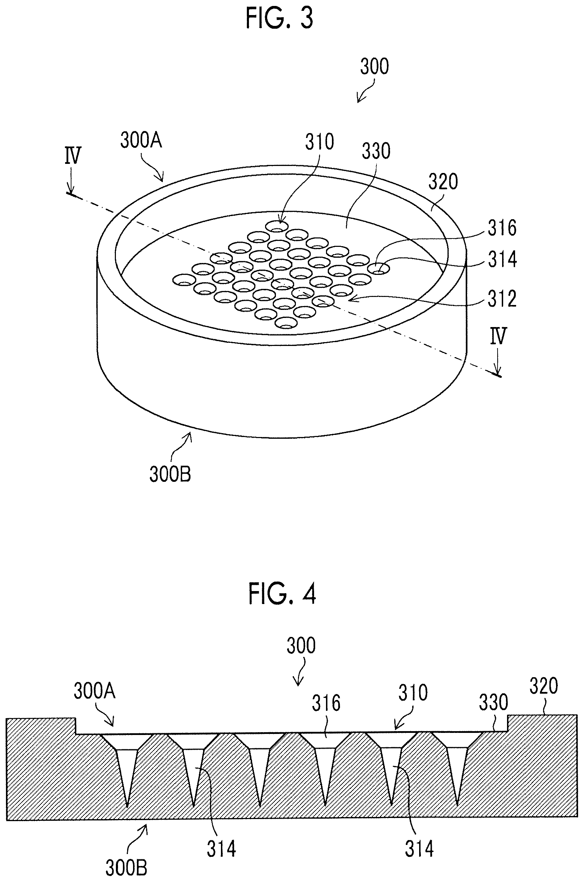

As illustrated in Figs. 3 and 4, in order to produce the microneedle array 100, a mold 300 is prepared. The mold 300 has a first surface 300A and a second surface 300B which oppose each other. A plurality of needle-like recessed portions 310 are formed in the first surface 300A of the mold 300.

The mold 300 comprises a weir 320 which has a certain height on the first surface 300A side where the needle-like recessed portions 310 are formed and surrounds the periphery of the needle-like recessed portions 310. The mold 300 can be made of, for example, a silicone resin. A flat surface 330 is provided between the needle-like recessed portions 310 and the weir 320. A recessed portion pattern 312 is constituted by the plurality of needle-like recessed portions 310.

The material of the mold 300 is desirably a material excellent in gas permeability such as a silicone resin, a thermoplastic resin, and a photocurable resin. Among the materials, a silicone resin is preferable.

The needle-like recessed portion 310 has an inverted shape of the needle-like protruding portion 112 of the microneedle array 100. The needle-like recessed portion 310 includes a distal end recessed portion 314 corresponding to the needle portion 114 and a cup portion 316 corresponding to the frustum portion 116. The flat surface 330 has a flat shape corresponding to the sheet portion 102 of the microneedle array 100.

The connecting portion between the flat surface 330 of the first surface 300A and the cup portion 316 is preferably chamfered (not illustrated). The radius of curvature of the chamfer is preferably 30 µm or more and 300 µm or less. As a result, it is possible to prevent the drug solution that has landed on the needle-like recessed portion 310 from adhering to an edge portion and entering a state of not moving. The drug solution can be easily collected or caused to flow into the center of the needle-like recessed portion 310, and thus fill the distal end of the needle-like recessed portion 310. The connecting portion is an example of the edge of the opening of the needle-like recessed portion 310.

The distal end recessed portion 314 has a tapered shape in a depth direction of the mold 300. The distal end recessed portion 314 can have a diameter of 150 µm or more and 500 µm or less and a height of 150 µm or more and 2000 µm or less. The cup portion 316 is provided with an opening in the first surface 300A of the mold 300, has a shape that narrows in the depth direction of the mold 300, and is connected to the distal end recessed portion 314 at the narrowest portion. The cup portion 316 can have a diameter of 600 µm or more and 1200 µm or less and a height of 100 µm or more and 500 µm or less.

The shape of the needle-like recessed portion 310 is not limited to this example. For example, any shape of a cone, a quadrangular pyramid, and a polygonal pyramid without the cup portion 316 can be applied. Moreover, a rocket shape provided with an intermediate recessed portion having a constant width in the depth direction, such as a cylinder, a quadrangular prism, or a polygonal column, between the distal end recessed portion 314 and the cup portion 316 may be applied. In addition, a through-hole that reaches the second surface 300B of the mold 300 and penetrates the mold 300 can be formed at the distal end of the distal end recessed portion 314. The arrangement, pitch, number, and the like of the needle-like recessed portions 310 are determined based on the arrangement, pitch, number, and the like of the needle-like protruding portions 112 necessary for the microneedle array 100.

The mold 300 can be produced using a plate precursor (not illustrated) that is a duplicate of the microneedle array 100. The plate precursor is produced by machining a metal substrate using a cutting tool such as a diamond tool. As the metal substrate, stainless steel, an aluminum alloy, Ni, or the like can be used.

The mold 300 in which the plurality of needle-like recessed portions 310 are formed can be produced by adding a silicone resin before being cured dropwise onto a prepared plate precursor, thereafter curing the silicone resin, and peeling the cured silicone resin from the plate precursor. The mold 300 made of the silicone resin may have gas permeability.

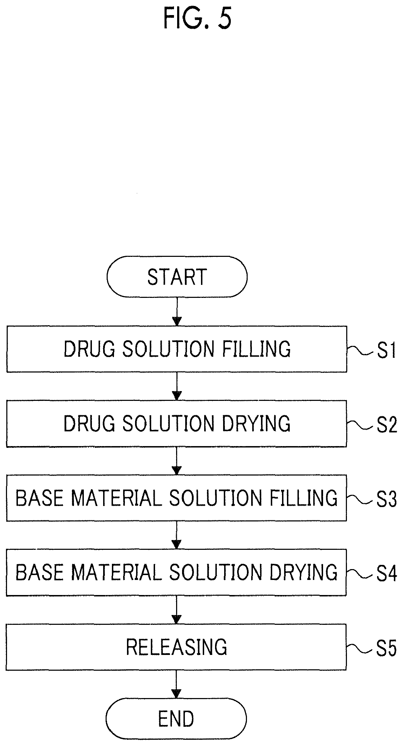

Fig. 5 is a flowchart showing each step of the manufacturing method of the microneedle array. The manufacturing method of the microneedle array 100 includes a drug solution filling step (step S1) of filling the needle-like recessed portion 310 of the mold 300 with the drug solution, a drug solution drying step (step S2) of drying the filled drug solution, a base material solution filling step (step S3) of filling the needle-like recessed portion 310 with a base material solution, a base material solution drying step (step S4) of drying the filled base material solution, and a releasing step (step S5) of releasing the formed microneedle array 100 from the mold 300.

### Drug Solution Filling Step (Step S1)

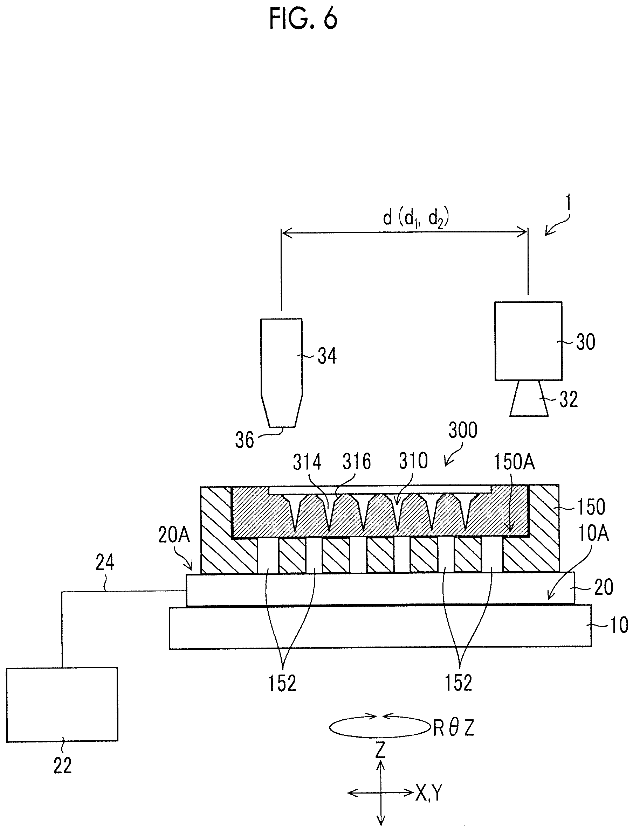

In the drug solution filling step, a liquid droplet of the drug solution is ejected from a nozzle 36 (see Fig. 6) of an ejection head 34 that ejects the drug solution toward the needle-like recessed portion 310 of the mold 300, and the mold 300 is suctioned by a suction pump 22 (see Fig. 6). Details of the drug solution filling step will be described later. The drug solution is made of, for example, a solution containing the first material M1 and the drug.

### Drug Solution Drying Step (Step S2)

In the drug solution drying step, for example, drying is performed by blowing air to the drug solution filling the needle-like recessed portion 310. The environment around the mold 300 may be reduced in pressure. However, the drying method is not particularly limited.

### Base Material Solution Filling Step (Step S3)

In the base material solution filling step, the needle-like recessed portion 310 is filled with the base material solution. The base material solution is made of a solution containing the second material M2. The second material M2 does not contain the drug.

Examples of a method of filling the needle-like recessed portion 310 with the base material solution include a filling method using a spin coater. The filling method is not particularly limited, and the base material solution is filled by a dispenser or the like.

### Base Material Solution Drying Step (Step S4)

In the base material solution drying step, as in the drug solution drying step, drying is performed by blowing air to the base material solution filling the needle-like recessed portion 310. However, the drying method is not particularly limited.

### Releasing Step (Step S5)

In the releasing step, the microneedle array 100 is released from the mold 300 by drying the drug solution and the base material solution.

### Drug Solution Filling Apparatus

Fig. 6 is a schematic configuration diagram of a drug solution filling apparatus 1 (an example of a manufacturing apparatus for a microneedle array) used in the drug solution filling step. The drug solution filling apparatus 1 includes an XYZ stage 10, an adsorption plate 20, the suction pump 22, a camera 30, the ejection head 34 that ejects the drug solution, and the like.

The XYZ stage 10 (an example of a positioning unit) has a placement surface 10A parallel to an XY plane. The XYZ stage 10 is provided so as to be movable by a motor (not illustrated) in an X direction and a Y direction orthogonal to the X direction, which are two directions parallel to the XY plane. The XYZ stage 10 is provided so as to be movable in a Z direction orthogonal to the XY plane. Furthermore, the XYZ stage 10 is provided so as to be movable in an RθZ direction, which is a rotation direction with the direction parallel to the Z direction as the rotation axis.

The XYZ stage 10 can apply vibration to the mold 300. Examples of the vibration include (1) a horizontal reciprocating motion parallel to the XY plane, (2) a horizontal circular motion parallel to the XY plane, (3) a vertical reciprocating motion parallel to the Z direction, (4) a combination of the horizontal reciprocating motion and the vertical reciprocating motion, and (5) a combination of the horizontal circular motion and the vertical reciprocating motion.

The adsorption plate 20 is fixed to the placement surface 10A of the XYZ stage 10. The adsorption plate 20 has a placement surface 20A parallel to the XY plane. The placement surface 20A is provided with a plurality of adsorption holes (not illustrated). The adsorption plate 20 may be made of a porous member.

The suction pump 22 is connected to the adsorption plate 20 via a suction pipe 24. By driving the suction pump 22, air can be suctioned from the plurality of adsorption holes (not illustrated) of the placement surface 20A of the adsorption plate 20.

A conveyance holding device 150 is placed on the placement surface 20A of the adsorption plate 20. In the conveyance holding device 150, the mold 300 is mounted on a placement surface 150A. Accordingly, the mold 300 can move in each direction as the XYZ stage 10 moves in the X direction, the Y direction, the Z direction, and the RθZ direction.

A plurality of adsorption holes 152 pass through the placement surface 150A of the conveyance holding device 150. By driving the suction pump 22, the second surface 300B (not illustrated) of the mold 300 is suctioned via the plurality of adsorption holes (not illustrated) of the placement surface 20A of the adsorption plate 20 and the plurality of adsorption holes 152 of the conveyance holding device 150.

The camera 30 comprises, in addition to an imaging lens 32, an imaging element (not illustrated), an analog-to-digital converter, and an image processing circuit.

The imaging lens 32 is a lens group comprising a zoom lens, a focus lens, and the like, and causes incidence ray from a subject to be incident onto the imaging element.

The imaging element is a charge coupled device (CCD) type imaging element or a complementary metal oxide semiconductor (CMOS) type imaging element in which a large number of light-receiving elements are two-dimensionally arranged on an imaging surface (not illustrated). The imaging element is disposed in a rear stage of an optical path of the incidence ray of the imaging lens 32.

The imaging lens 32 forms an image of the incidence ray on an imaging surface of the imaging element. The imaging element outputs an analog imaging signal corresponding to the amount of received light. This imaging signal is converted into a digital signal by the analog-to-digital converter, and then generated into an image signal by the image processing circuit.

The camera 30 is disposed above the XYZ stage 10 in the Z direction, and the imaging lens 32 is directed downward in the Z direction. Accordingly, the camera 30 can image the mold 300 placed on the XYZ stage 10.

The ejection head 34 is disposed at a position above the XYZ stage 10 in the Z direction and separated from the camera 30 by a distance d on the XY plane including a distance d₁ in the X direction and a distance d₂ in the Y direction. The ejection head 34 includes the nozzle 36 (an example of a drug solution ejection nozzle) that ejects liquid droplets of the drug solution in the first direction. Here, the nozzle 36 is directed downward in the Z direction, and the first direction is a downward direction in the Z direction. The ejection head 34 illustrated in Fig. 6 includes one nozzle 36, but may include a plurality of nozzles 36.

As the ejection head 34, for example, an ink jet head such as a solenoid type ink jet head or a piezoelectric ink jet head can be used. The amount of one liquid droplet ejected from the nozzle 36 is preferably 1 nL to 150 nL, and more preferably 45 nL to 80 nL.

In a case where a required amount of the drug solution is ejected from the nozzle 36 into the needle-like recessed portion 310, one liquid droplet or a plurality of liquid droplets can be ejected. There is no limitation on the number of liquid droplets to be ejected as long as a required amount of the drug solution can be filled. In the case of ejecting a plurality of liquid droplets, as the number of ejected liquid droplets increases, the liquid droplet amount of one liquid droplet decreases.

The drug solution ejected from the nozzle 36 flies downward in the Z direction and lands on an object (in this case, the mold 300). Therefore, the position of the nozzle 36 on the XY plane and the position on the XY plane where the drug solution lands are the same.

The drug solution contains a drug stock solution, a sugar, an additive, and the like as the drug. Moreover, the drug solution contains water, ethanol, or the like as a solvent.

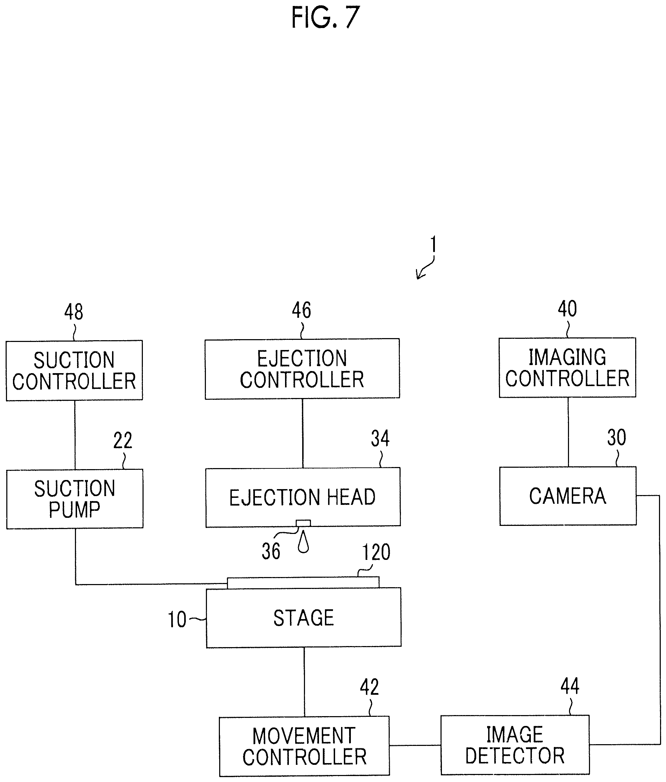

Fig. 7 is a block diagram illustrating an electrical configuration of the drug solution filling apparatus 1. The drug solution filling apparatus 1 includes an imaging controller 40, a movement controller 42, an image detector 44, an ejection controller 46, a suction controller 48, and the like.

The imaging controller 40 causes the camera 30 to capture an image.

The movement controller 42 controls a relative movement between the mold 300 placed on the XYZ stage 10 and the ejection head 34. Here, the mold 300 is moved by driving the XYZ stage 10, but the ejection head 34 may be moved, or both the mold 300 and the ejection head 34 may be moved.

The movement controller 42 applies vibration selected from (1) the horizontal reciprocating motion parallel to the XY plane, (2) the horizontal circular motion parallel to the XY plane, (3) the vertical reciprocating motion parallel to the Z direction, (4) the combination of the horizontal reciprocating motion and the vertical reciprocating motion, and (5) the combination of the horizontal circular motion and the vertical reciprocating motion, to the mold 300 placed on the XYZ stage 10.

The image detector 44 detects the position of the mold 300 based on the image of the mold 300 captured by the camera 30. In the present embodiment, the position of the needle-like recessed portion 310 is detected by recognizing the needle-like recessed portion 310 from the image of the mold 300.

The ejection controller 46 controls the timing of ejecting the drug solution from the nozzle 36, and the liquid droplet amount of the drug solution to be ejected, by controlling the ejection head 34.

The suction controller 48 controls the presence or absence of suction by the suction pump 22.

### Drug Solution Filling Step

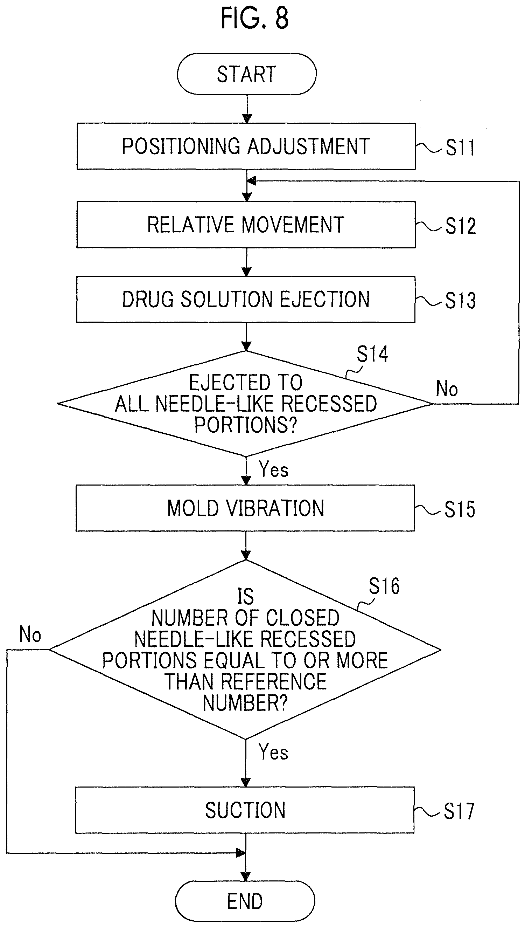

Fig. 8 is a flowchart showing each step included in the drug solution filling step. The drug solution filling step includes a positioning adjustment step (step S11), a relative movement step (step S12), a drug solution ejection step (step S13), an ejection finish determination step (step S14), a vibration step (step S15), an acceptance determination step (step S16), and a suction step (step S17).

### Positioning Adjustment Step (Step S11)

In the positioning adjustment step, the positioning is adjusted so that the position of the needle-like recessed portion 310 of the mold 300 placed on the XYZ stage 10 and the position of the nozzle 36 of the ejection head 34 coincide with each other. The position of the needle-like recessed portion 310 and the position of the nozzle 36 may coincide with each other so that the drug solution ejected from the nozzle 36 toward the needle-like recessed portion 310 lands on the needle-like recessed portion 310, and the positions of the two do not need to strictly coincide with each other. Here, the needle-like recessed portion 310 and the nozzle 36 are virtually positioned by detecting the position of the needle-like recessed portion 310 from the captured image.

First, the conveyance holding device 150 on which the mold 300 is mounted is placed on the placement surface 20A of the adsorption plate 20.

The movement controller 42 controls the XYZ stage 10 to move the mold 300 within the angle of view of the captured image of the camera 30. The imaging controller 40 controls the camera 30 to capture an image of the mold 300 (an example of an imaging step). The image detector 44 calculates the position of each needle-like recessed portion 310 by analyzing the image of the mold 300 captured by the camera 30.

For example, the needle-like recessed portion 310 of the mold 300 is moved to the center within the angle of view of the captured image of the camera 30 by the XYZ stage 10, and the XY plane coordinates (X,Y) of the XYZ stage 10 at this point are detected. By performing this for all the needle-like recessed portions 310, the positions of all the needle-like recessed portions 310 can be detected.

In the image of the mold 300 captured by the camera 30, the flat surface 330 has a relatively bright brightness, and the needle-like recessed portion 310 has a relatively dark brightness. By using this contrast, the needle-like recessed portion 310 can be moved to the center within the angle of view of the captured image of the camera 30.

Instead of moving all the needle-like recessed portions 310 to the center within the angle of view of the image captured by the camera 30, only the XY plane coordinates (X,Y) of three to five needle-like recessed portions 310 may be detected and the direction (rotation) of the mold 300 in the XY plane from the coordinates and the deviation or expansion and contraction of the mold 300 in the XY plane may be analyzed to detect the positions of the other needle-like recessed portions 310.

Alternatively, the mold 300 may be provided with a plurality of alignment marks, and the XY plane coordinates (X,Y) of the needle-like recessed portion 310 may be detected by reading the alignment marks.

As described above, by detecting the position of the needle-like recessed portion 310 based on the XY plane coordinates (X,Y) of the XYZ stage 10, the needle-like recessed portion 310 and the nozzle 36 are virtually positioned. Mechanical positioning may be performed by a positioning adjustment holding device or the like.

Furthermore, the position (height) of the mold 300 in the Z direction may be adjusted by measuring the distance between the needle-like recessed portion 310 or the alignment mark and the camera 30. The distance between the nozzle 36 and the mold 300 is preferably adjusted to be 0.5 mm to 5 mm, and preferably 1 mm to 2 mm.

### Relative Movement Step (Step S12)

The movement controller 42 controls the XYZ stage 10 based on the detection result of the image detector 44 to move the mold 300 in the X direction and the Y direction so as to cause the position of the nozzle 36 of the ejection head 34 on the XY plane and the position of the needle-like recessed portion 310 on the XY plane to coincide with each other. That is, the position of the nozzle 36 and the position of the needle-like recessed portion 310 are caused to coincide with each other in a plan view in the direction (Z direction) parallel to the ejection direction of the drug solution from the nozzle 36.

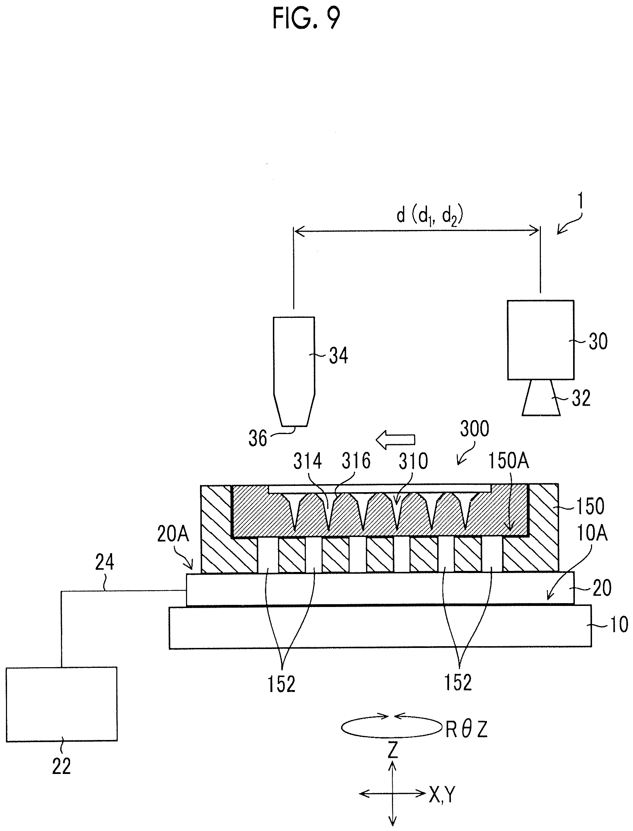

Coordinates (X + d₁, Y + d₂) obtained by adding the distance d₁ in the X direction between the camera 30 and the nozzle 36 of the ejection head 34 and the distance d₂ in the Y direction to the coordinates (X,Y) of the needle-like recessed portion 310 calculated in step S11 are the coordinates of the nozzle 36. As illustrated in Fig. 9, the movement controller 42 moves the XYZ stage 10 to the coordinates.

### Drug Solution Ejection Step (Step S13)

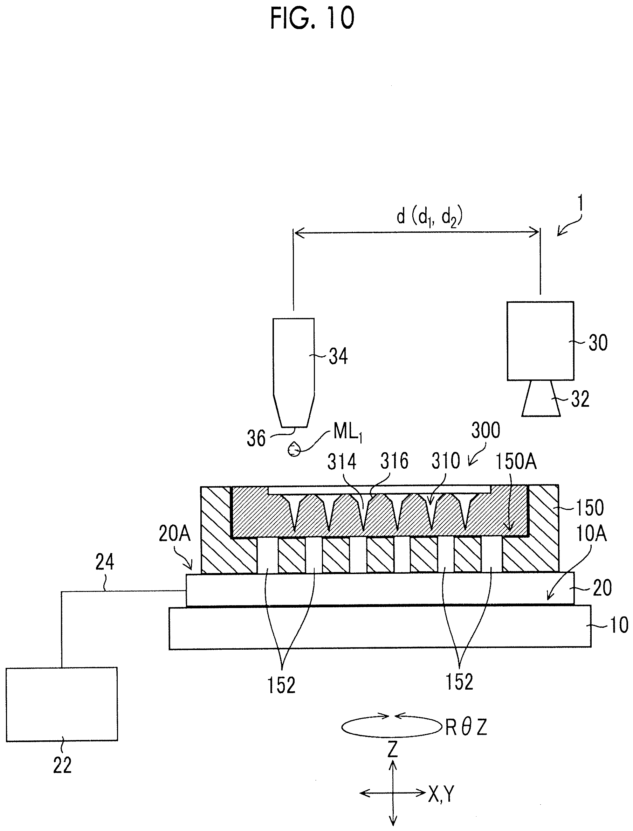

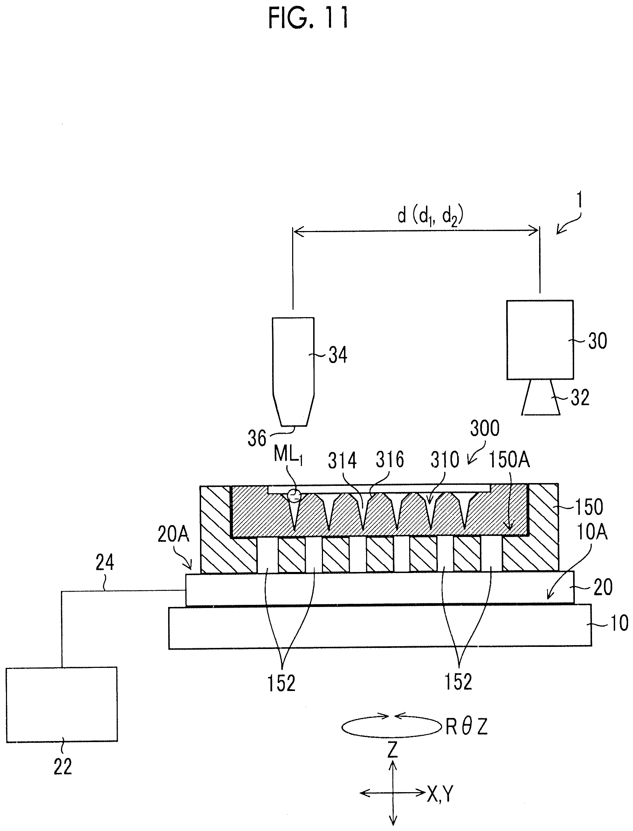

As illustrated in Fig. 10, the ejection controller 46 controls the ejection head 34 to eject a drug solution ML₁ from the nozzle 36. As illustrated in Fig. 11, the ejected drug solution ML₁ lands on the needle-like recessed portion 310. Here, one droplet of the drug solution ML₁ is ejected from the nozzle 36 to one needle-like recessed portion 310 and is caused to land on the needle-like recessed portion 310. A plurality of droplets of the drug solution ML₁ may be caused to land on one needle-like recessed portion 310.

### Ejection Finish Determination Step (Step S14)

The ejection controller 46 determines whether or not the drug solution ML₁ has been ejected to land on all the needle-like recessed portions 310 of the mold 300. Here, the number of ejections of the drug solution ejected in the drug solution ejection step and the number of needle-like recessed portions 310 whose positions have been detected in the positioning adjustment step are compared to each other for determination.

In a case where it is determined that there is a needle-like recessed portion 310 on which the drug solution ML₁ does not land, the process returns to step S12 and the same processing is performed. That is, the position on the XY plane of the needle-like recessed portion 310 to which the drug solution ML₁ has not been ejected and the position on the XY plane of the nozzle 36 are caused to coincide with each other (step S12), and the drug solution is ejected from the nozzle 36 to land on the needle-like recessed portion 310 (step S13). The ejection order of the drug solution to the needle-like recessed portions 310 is not particularly limited, but from the viewpoint of shortening the total movement distance of the XYZ stage 10, it is preferable to eject the drug solution sequentially in order from the needle-like recessed portion 310 disposed at the end of the mold 300 to adjacent needle-like recessed portions 310.

In a case where it is determined that the drug solution ML₁ has landed on all the needle-like recessed portions 310, the process proceeds to step S15.

### Vibration Step (Step S15)

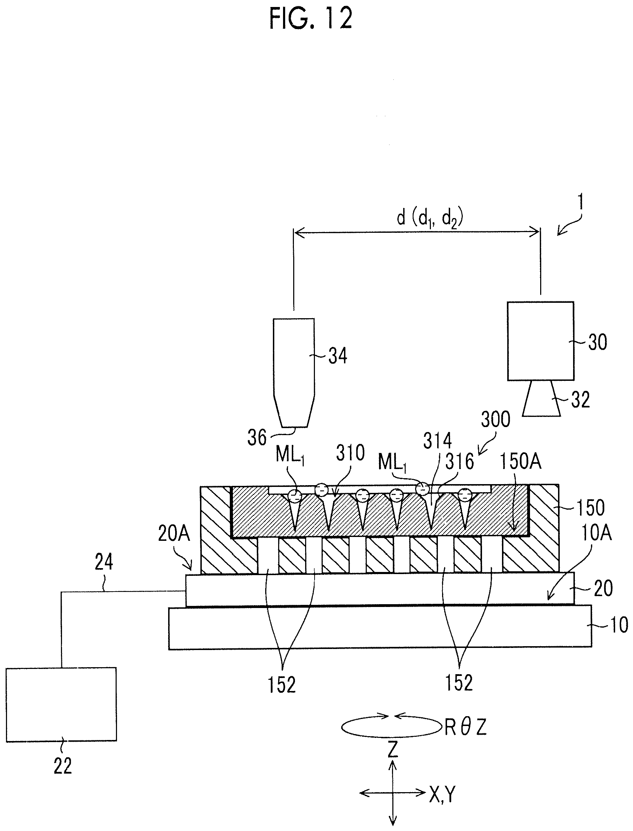

Fig. 12 illustrates the mold 300 having finished the ejection of the drug solution ML₁ to the needle-like recessed portion 310. The drug solution ML₁ ejected in the drug solution ejection step needs to close the needle-like recessed portion 310, that is, be into contact with the entire circumference of the wall portion of the needle-like recessed portion 310. In a case where the landed drug solution ML₁ does not close the needle-like recessed portion 310, the drug solution ML₁ landed in the suction step cannot fill the distal end of the tapered shape of the distal end recessed portion 314.

Therefore, when the position of the nozzle 36 and the position of the needle-like recessed portion 310 are caused to coincide with each other in the relative movement step, precise positional accuracy is required. For this reason, it is necessary to precisely adjust the positioning in the positioning adjustment step.

However, in Fig. 12, a plurality of the drug solutions ML₁ do not close the needle-like recessed portions 310. Even in a case where the positioning is precisely adjusted, a positional deviation is caused by a landing positional deviation due to apparatus vibration that occurs when the XYZ stage 10 or the nozzle 36 is moved at a high velocity, a landing positional deviation due to a change in liquid droplet flight velocity caused by a state change in the nozzle 36 over time and a change in the drug solution ML₁ over time, a positional deviation during the installation of the mold 300, a positioning deviation of the needle-like recessed portions 310 due to expansion and contraction or strain of the mold 300 itself, and combinations thereof. It is difficult to completely eliminate the positional deviation.

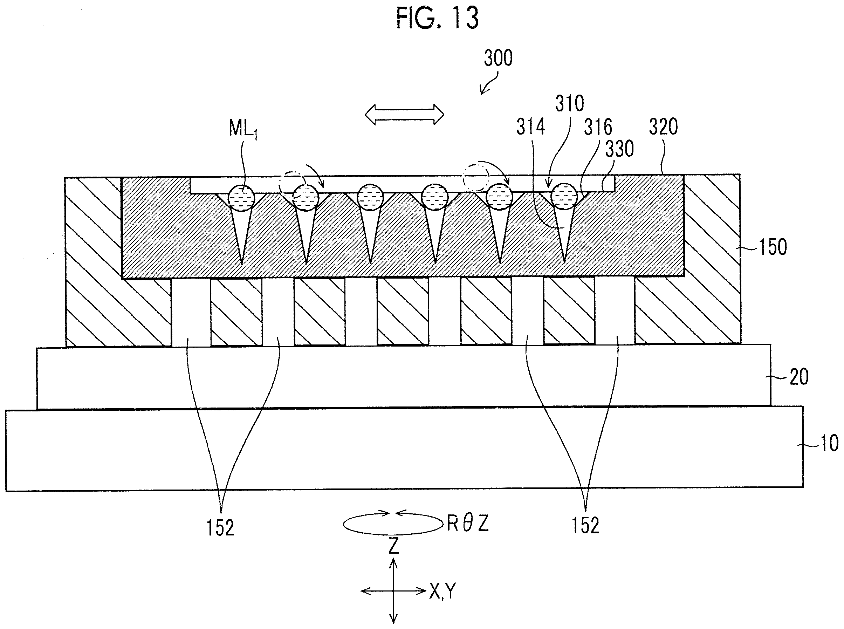

Therefore, in the present embodiment, as illustrated in Figs. 8 and 13, after the ejection is finished, the mold 300 is vibrated to move the drug solution ML₁ toward the needle-like recessed portion 310, whereby the needle-like recessed portion 310 is closed with the drug solution ML₁. As illustrated in Fig. 13, the vibration is the horizontal reciprocating motion parallel to the XY plane.

The horizontal reciprocating motion is preferably performed, for example, with an amplitude of 1 mm or more and 10 mm or less, a frequency of 15 Hz or higher and 100 Hz or lower, and a time of 1 second or longer and 180 seconds or shorter. The horizontal reciprocating motion within the ranges of amplitude, frequency, and time can move the drug solution ML₁ and close the needle-like recessed portion 310 with the drug solution ML₁. The amplitude is more preferably 3 mm or more and 8 mm or less, the frequency is more preferably 40 Hz or higher and 70 Hz or lower, and the time is more preferably 50 seconds or longer and 80 seconds or shorter.

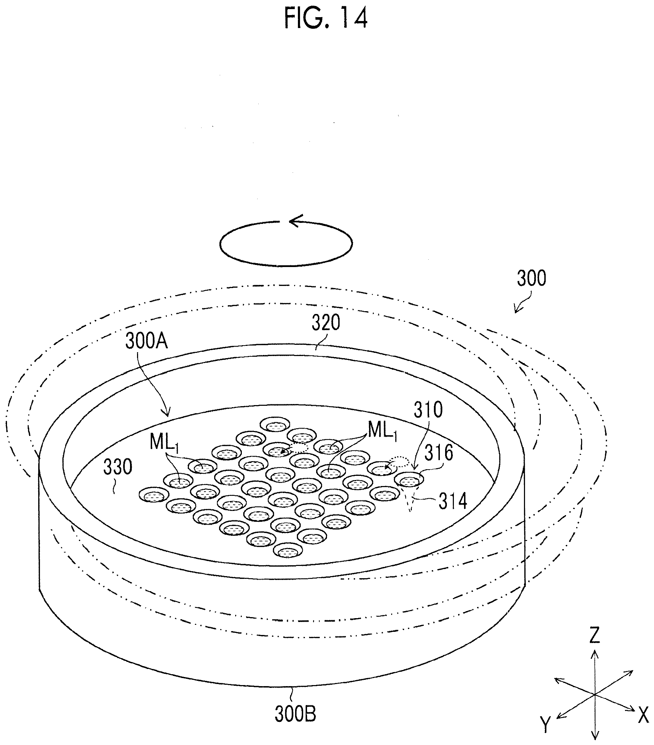

The vibration is not limited to the horizontal reciprocating motion illustrated in Fig. 13. For example, as illustrated in Fig. 14, the vibration is the horizontal circular motion parallel to the XY plane. The horizontal circular motion is preferably performed, for example, with an amplitude of 1 mm or more and 10 mm or less, a frequency of 15 Hz or higher and 100 Hz or lower, and a time of 1 second or longer and 180 seconds or shorter. The horizontal circular motion within the ranges of amplitude, frequency, and time can move the drug solution ML₁ and close the needle-like recessed portion 310 with the drug solution ML₁. The amplitude is more preferably 3 mm or more and 8 mm or less, the frequency is more preferably 40 Hz or higher and 70 Hz or lower, and the time is more preferably 50 seconds or longer and 80 seconds or shorter.

The horizontal circular motion can be realized, for example, by circularly moving the mold 300 around a rotation axis (parallel to the Z direction) that is eccentric from the center axis (parallel to the Z direction) of the mold 300. The circular motion may be a true circular locus or an elliptical locus.

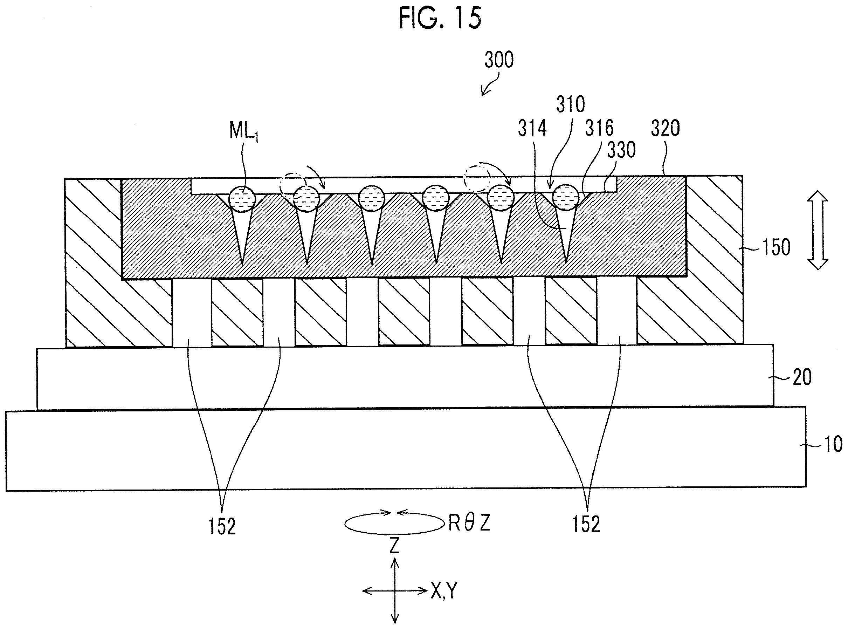

The vibration is, for example, as illustrated in Fig. 15, the vertical reciprocating motion parallel to the Z direction. The vertical reciprocating motion is preferably performed, for example, with an amplitude of 1 mm or more and 10 mm or less, a frequency of 15 Hz or higher and 100 Hz or lower, and a time of 1 second or longer and 180 seconds or shorter. The vertical reciprocating motion within the ranges of amplitude, frequency, and time can move the drug solution ML₁ and close the needle-like recessed portion 310 with the drug solution ML₁. The amplitude is more preferably 3 mm or more and 8 mm or less, the frequency is more preferably 40 Hz or higher and 70 Hz or lower, and the time is more preferably 50 seconds or longer and 80 seconds or shorter.

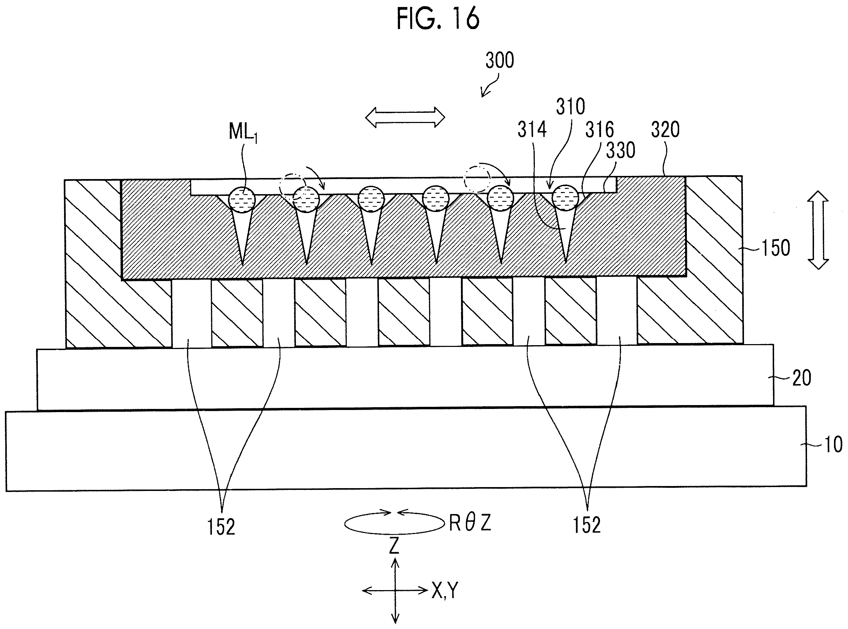

The vibration is, for example, as illustrated in Fig. 16, the combination of the horizontal reciprocating motion and the vertical reciprocating motion. Each of the horizontal reciprocating motion and the vertical reciprocating motion is preferably performed, for example, with an amplitude of 1 mm or more and 10 mm or less, a frequency of 15 Hz or higher and 100 Hz or lower, and a time of 1 second or longer and 180 seconds or shorter. Each of the horizontal reciprocating motion and the vertical reciprocating motion within the ranges of amplitude, frequency, and time can move the drug solution ML₁ and close the needle-like recessed portion 310 with the drug solution ML₁. The amplitude is more preferably 3 mm or more and 8 mm or less, the frequency is more preferably 40 Hz or higher and 70 Hz or lower, and the time is more preferably 50 seconds or longer and 80 seconds or shorter.

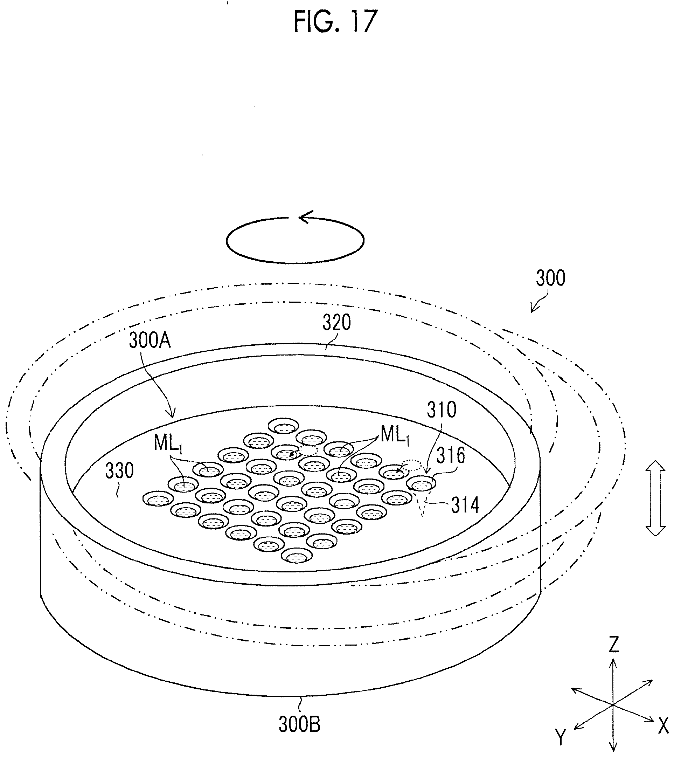

Furthermore, for example, as illustrated in Fig. 17, the vibration is the combination of the horizontal circular motion and the vertical reciprocating motion. Each of the horizontal circular motion and the vertical reciprocating motion is preferably performed, for example, with an amplitude of 1 mm or more and 10 mm or less, a frequency of 15 Hz or higher and 100 Hz or lower, and a time of 1 second or longer and 180 seconds or shorter. Each of the horizontal circular motion and the vertical reciprocating motion within the ranges of amplitude, frequency, and time can move the drug solution ML₁ and close the needle-like recessed portion 310 with the drug solution ML₁. The amplitude is more preferably 3 mm or more and 8 mm or less, the frequency is more preferably 40 Hz or higher and 70 Hz or lower, and the time is more preferably 50 seconds or longer and 80 seconds or shorter.

### Acceptance Determination Step (Step S16)

In the acceptance determination step, after finishing the vibration step, the imaging controller 40 and the camera 30 image all the needle-like recessed portions 310 of the mold 300, and check whether or not the number of the needle-like recessed portions 310 whose openings are closed by the drug solution is equal to or more than a predetermined reference number. This reference number is determined by the minimum number of needle-like protruding portions 112 required in one microneedle array 100.

In a case where the number of closed needle-like recessed portions 310 is less than the reference number, the process of this flowchart is finished as a rejected product. In a case where the number of closed needle-like recessed portions 310 is equal to or more than the reference number, the process proceeds to step S17 as an acceptable product.

### Suction Step (Step S17)

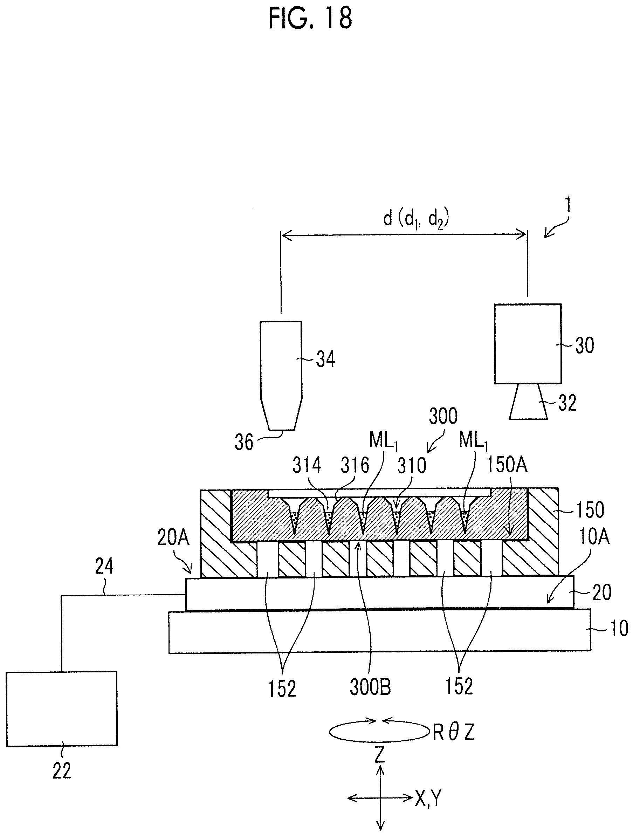

The suction controller 48 drives the suction pump 22 to suction the second surface 300B of the mold 300. By this suction, as illustrated in Fig. 18, the drug solution ML₁ that has landed on the needle-like recessed portion 310 fills the distal end of the tapered shape of the distal end recessed portion 314.

As above, the drug solution filling step is finished. In the embodiment, the case where the drug solution ejection step, the vibration step, and the suction step are performed with the same XYZ stage 10 has been described. However, the embodiment is not limited to this form. The drug solution ejection step, the vibration step, and the suction step can be performed with separate stages.

For example, when the drug solution ejection step is finished, the conveyance holding device 150 on which the mold 300 is mounted is moved to a vibration stage that performs the vibration step, and the mold 300 is vibrated by the vibration stage. Next, when the vibration step is finished, the conveyance holding device 150 on which the mold 300 is mounted is moved to a suction stage that performs the suction step, and the second surface 300B of the mold 300 is suctioned by the suction stage. The acceptance determination step can be performed by either the vibration stage or the suction stage.

Here, the distance d₁ and the distance d₂ are treated as known values, but in a case where the distances are unknown, the distances can be obtained as follows.

A dummy mold that is not provided with the needle-like recessed portions 310 is mounted on the conveyance holding device 150 and placed on the placement surface 20A of the adsorption plate 20. The drug solution is ejected from the nozzle 36 to the dummy mold so as to land on the dummy mold.

Next, the XYZ stage 10 is moved in the X direction and the Y direction so that the landed drug solution is disposed at the center of the angle of view of the captured image of the camera 30. Here, the amount of movement of the XYZ stage 10 in the X direction is the distance d₁, and the amount of movement thereof in the Y direction is the distance d₂.

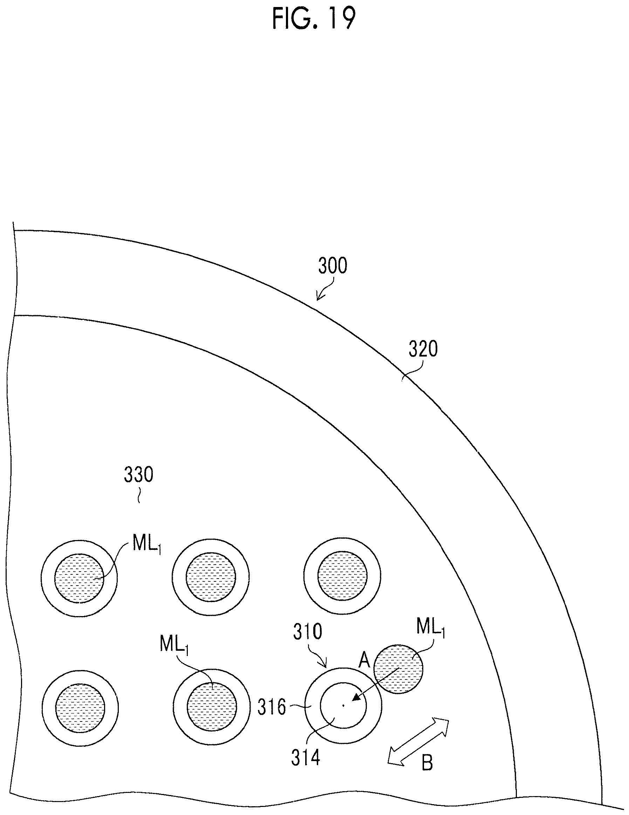

Next, a preferable form in the vibration step (step S15) will be described. For example, in a case where the vibration is the horizontal reciprocating motion, as illustrated in Fig. 19, it is preferable that the movement direction (arrow A) of the drug solution ML₁ and the direction of the horizontal reciprocating motion (arrow B) are the same direction. The same direction may be achieved when the movement direction of the drug solution ML₁ and the direction of the horizontal reciprocating motion are parallel or substantially parallel in a plan view of the mold 300. By causing the movement direction of the drug solution ML₁ and the direction of the horizontal reciprocating motion to be the same direction, the drug solution ML₁ can be moved to the needle-like recessed portion 310 more reliably within a short period of time.

As the movement direction of the drug solution ML₁, for example, a direction that is easily deviated can be obtained in advance from the characteristics of the apparatus or the like. The movement direction of the drug solution ML₁ is obtained by, for example, imaging the needle-like recessed portion 310 that is not closed by the drug solution ML₁ and the drug solution ML₁ that is not closing the needle-like recessed portion 310 by the imaging device, and analyzing the captured image. The vibration of the horizontal reciprocating motion in the same direction as the obtained movement direction of the drug solution ML₁ is applied to the mold 300.

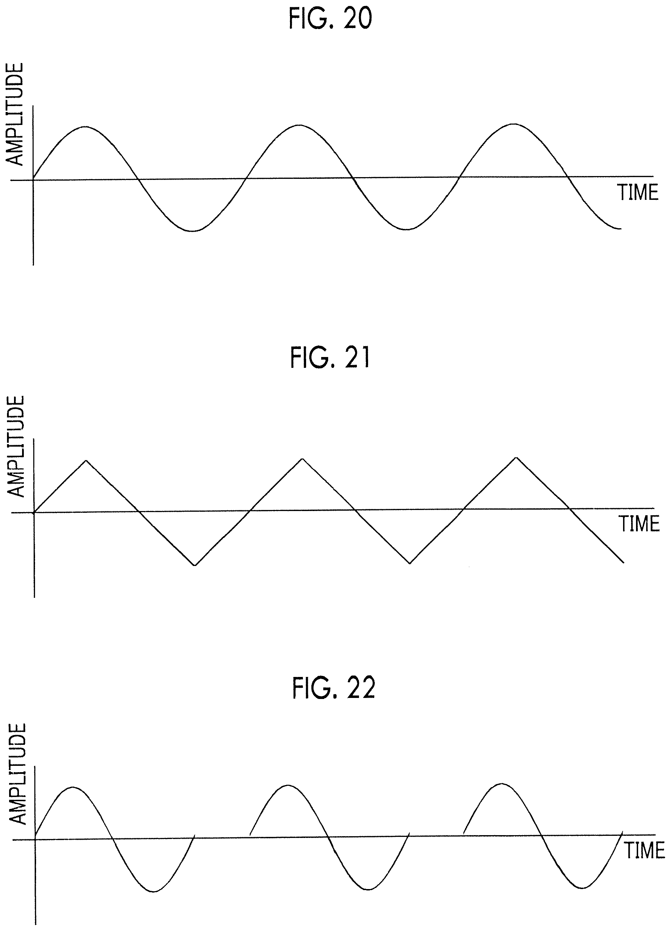

The vibration is not particularly limited as long as the vibration can be defined by the maximum amplitude, frequency, and time. Figs. 20 to 22 are waveform diagrams in which the vertical axis represents amplitude and the horizontal axis represents time. Fig. 19 shows a sine wave, Fig. 21 shows a triangle wave, and Fig. 22 shows a pulse wave. However, the vibration is not limited to the waveform diagrams shown in Figs. 20 to 22.

### Examples

The present invention will be specifically described by the following examples, but the present invention is not limited to these examples.

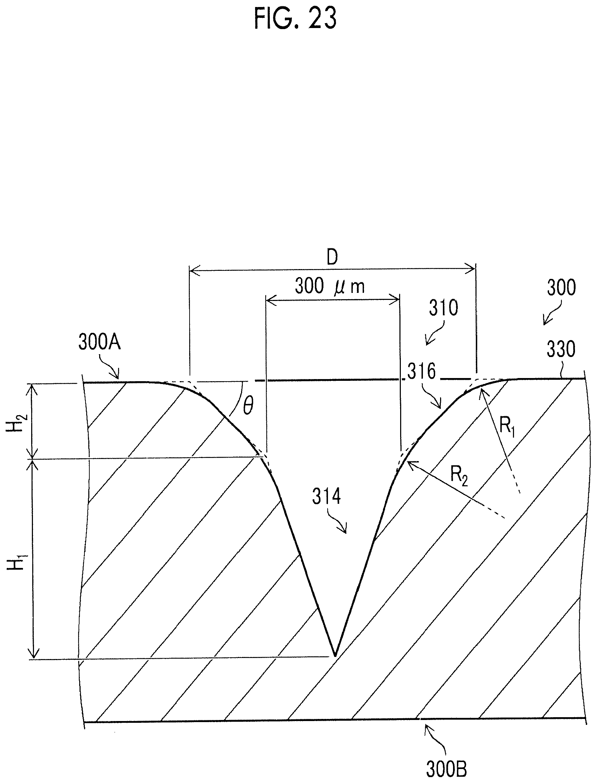

First, a mold 300 in which 100 needle-like recessed portions 310 were formed was prepared. Fig. 23 is a cross-sectional view for describing the shape of the needle-like recessed portion 310 according to the example, and is a further enlarged view of Fig. 3. As illustrated in Fig. 23, the diameter of a distal end recessed portion 314 is 300 µm. In addition, a height (depth) H₁ was set to 720 µm, and an opening diameter (opening diameter of the needle-like recessed portion 310) D of a cup portion 316 was set to 600 µm. An angle θ of an inner portion of the mold 300, which is the angle formed between a flat surface 330 of the mold 300 and the inside of the cup portion 316, was set to 45°. The height (depth) H₂ of the cup portion 316 was 150 µm from the opening diameter D and the angle θ.

Furthermore, the connecting portion between the flat surface 330 of the first surface 300A and the cup portion 316 was chamfered, and the radius of curvature R₁ of the chamfered arc was set to 50 µm. Due to the chamfering, the angle θ was an angle formed by an extension line of the flat surface 330 of the first surface 300A and an extension line of the cup portion 316.

Moreover, the connecting portion between the cup portion 316 and the distal end recessed portion 314 was chamfered, and the radius of curvature R₂ of the chamfered arc was set to 50 µm.

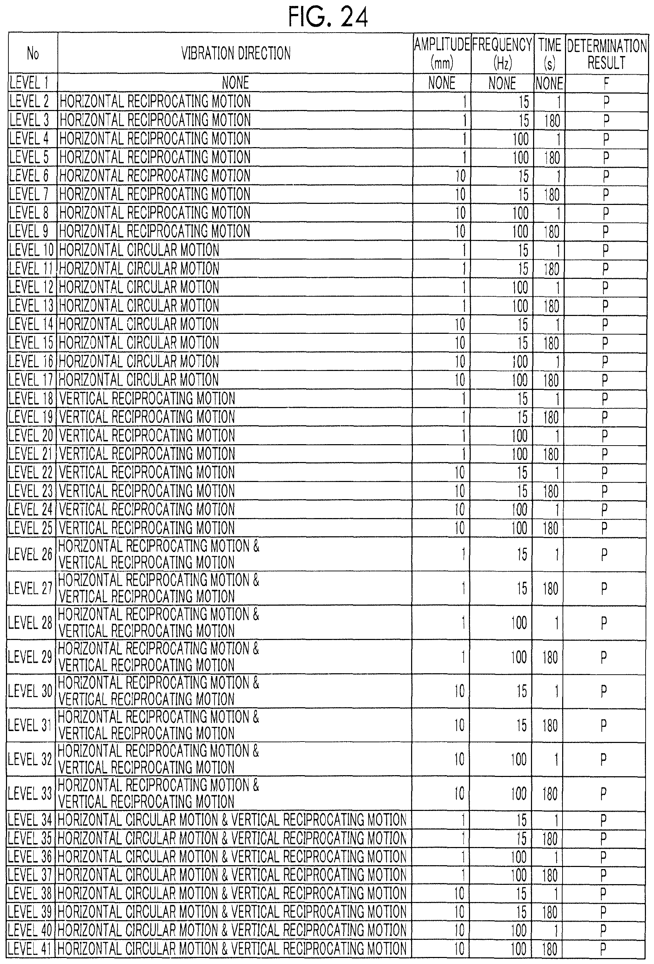

The ejection amount of a drug solution ejected from the nozzle 36 was 35 nL, and the drop velocity was 0.3 m/s. At each level with the kind of vibration, amplitude, frequency, and time as parameters, whether or not the needle-like recessed portion 310 could be closed and filled with the drug solution was evaluated. Fig. 24 is a table showing the parameters at each level and the evaluation results. As shown in Fig. 24, Levels 1 to 41 were evaluated.

### Determination Standard

A case where the ejected drug solution closed and filled all the 100 needle-like recessed portions 310 formed in the mold 300 was regarded as a non-defective product, and a case where one or more needle-like recessed portions 310 could not be closed and filled was regarded as a defective product. 100 molds 300 were prepared, and the drug solution was ejected to the 100 molds 300. A case of a non-defective product yield of 95% or more was determined as P, and a case of a non-defective product yield of less than 95% was determined as F.

In Level 1, since no vibration was applied to the mold 300, a determination result of "F" was obtained.

In Levels 2 to 9, vibration of a horizontal reciprocating motion was applied to the mold 300, and the amplitude, frequency, and time were changed. In each of Levels 2 to 9, a determination result of "P" was obtained.

In Levels 10 to 17, vibration of a horizontal circular motion was applied to the mold 300, and the amplitude, frequency, and time were changed. In each of Levels 10 to 17, a determination result of "P" was obtained.

In Levels 18 to 25, vibration of a vertical reciprocating motion was applied to the mold 300, and the amplitude, frequency, and time were changed. In each of Levels 18 to 25, a determination result of "P" was obtained.

In Levels 26 to 33, vibration of a combination of the horizontal reciprocating motion and the vertical reciprocating motion was applied to the mold 300, and the amplitude, frequency, and time were changed. In each of Levels 26 to 33, a determination result of "P" was obtained.

In Levels 34 to 41, vibration of a combination of the horizontal circular motion and the vertical reciprocating motion was applied to the mold 300, and the amplitude, frequency, and time were changed. In each of Levels 34 to 41, a determination result of "P" was obtained.

From the table in Fig. 24, it can be understood that the step of applying vibration to the mold 300 is useful for closing and filling the needle-like recessed portions 310 when the drug solution is ejected onto the mold 300.

### Others

The technical scope of the present invention is not limited to the scope described in the above embodiment. The configurations and the like in the embodiments can be appropriately combined between the embodiments without departing from the gist of the present invention.

### Explanation of References

1: drug solution filling apparatus
10: XYZ stage
10A: placement surface
20: adsorption plate
20A: placement surface
22: suction pump
24: suction pipe
30: camera
32: imaging lens
34: ejection head
36: nozzle
40: imaging controller
42: movement controller
44: image detector
46: ejection controller
48: suction controller
100: microneedle array
102: sheet portion
102A: first surface
102B: second surface
110: array pattern
112: needle-like protruding portion
114: needle portion
116: frustum portion
150: conveyance holding device
150A: placement surface
152: adsorption hole
300: mold
300A: first surface
300B: second surface
310: needle-like recessed portion
312: recessed portion pattern
314: distal end recessed portion
316: cup portion
320: weir
330: flat surface
A, B: arrow
d: distance
D: opening diameter
d₁: distance
d₂: distance
M1: first material
M2: second material
ML₁: drug solution
R₁, R₂: radius of curvature
S1, S2, S3, S4, S5, S11, S12, S13, S14, S15, S16, S17: step

## Claims

1. A manufacturing method of a microneedle array comprising:
an adjustment step of positioning and adjusting a mold having a first surface and a second surface, which oppose each other, and a plurality of needle-like recessed portions in the first surface, and an ejection nozzle which ejects a drug solution in a first direction;
a relative movement step of moving the mold and the ejection nozzle relative to each other to cause a position of the needle-like recessed portion and a position of the ejection nozzle to coincide with each other in a plan view in the first direction;
an ejection step of ejecting the drug solution from the ejection nozzle toward the needle-like recessed portion;
a vibration step of vibrating the mold to move the drug solution toward the needle-like recessed portion and close the needle-like recessed portion with the drug solution after the ejection step; and
a suction step of suctioning the second surface of the mold.

2. The manufacturing method of a microneedle array according to claim 1,
wherein the vibration in the vibration step is a horizontal reciprocating motion.

3. The manufacturing method of a microneedle array according to claim 2,
wherein a movement direction of the drug solution and a direction of the horizontal reciprocating motion are the same direction.

4. The manufacturing method of a microneedle array according to claim 1,
wherein the vibration in the vibration step is a horizontal circular motion.

5. The manufacturing method of a microneedle array according to claim 1,
wherein the vibration in the vibration step is a vertical reciprocating motion.

6. The manufacturing method of a microneedle array according to claim 2 or 3,
wherein the vibration in the vibration step further includes a vertical reciprocating motion.

7. The manufacturing method of a microneedle array according to claim 4,
wherein the vibration in the vibration step further includes a vertical reciprocating motion.

8. The manufacturing method of a microneedle array according to any one of claims 1 to 7,
wherein the needle-like recessed portion comprises:
a cup portion provided in the first surface of the mold; and
a distal end recessed portion which is connected to the cup portion and has a tapered shape in a depth direction of the mold,
an edge portion of an opening of the needle-like recessed portion is chamfered, and
a chamfer of the edge portion has a radius of curvature of 30 µm or more and 300 µm or less.

9. The manufacturing method of a microneedle array according to any one of claims 1 to 8,
wherein the vibration in the vibration step has an amplitude of 1 mm or more and 10 mm or less, a frequency of 15 Hz or higher and 100 Hz or lower, and a time of 1 second or longer and 180 seconds or shorter.

10. The manufacturing method of a microneedle array according to any one of claims 1 to 9, further comprising:
after the suction step, a drug solution drying step of drying the drug solution filling the needle-like recessed portion;
after the drug solution drying step, a base material solution filling step of filling the mold with a base material solution; and
a base material solution drying step of drying the filled base material solution.

## Patentansprüche

1. Fertigungsverfahren für eine Mikronadelanordnung, umfassend:
einen Justierschritt des Positionierens und Justierens einer Form, die eine erste Oberfläche und eine zweite Oberfläche, die einander abgewandt sind, aufweist, ferner mehrere nadelähnliche vertiefte Abschnitte in der ersten Oberfläche, und eine Ausstoßdüse, die eine Arzneimittellösung in einer ersten Richtung ausstößt;
einen Relativbewegungsschritt des Bewegens der Form und der Ausstoßdüse relativ zueinander, um zu veranlassen, dass eine Stelle des nadelähnlichen vertieften Abschnitts und eine Stelle der Ausstoßdüse in einer Draufsicht in der ersten Richtung miteinander überstimmen;
einen Ausstoßschritt des Ausstoßens der Arzneimittellösung aus der Ausstoßdüse in Richtung auf den nadelähnlichen vertieften Abschnitt;
einen Vibrationsschritt des Vibrierens der Form, um die Arzneimittellösung zu dem nadelähnlichen vertieften Abschnitt zu bewegen und den nadelähnlichen vertieften Abschnitt nach dem Ausstoßschritt mit der Arzneimittellösung zu verschließen; und
einen Saugschritt des Ansaugens der zweiten Oberfläche der Form.

2. Verfahren nach Anspruch 1,
bei dem die Vibration in dem Vibrationsschritt eine horizontale Hin- und Herbewegung ist.

3. Verfahren nach Anspruch 2,
bei dem eine Bewegungsrichtung der Arzneimittellösung und eine Richtung der horizontalen Hin- und Herbewegung die gleiche Richtung sind.

4. Verfahren nach Anspruch 1,
bei dem die Vibration in dem Vibrationsschritt eine horizontale kreisförmige Bewegung ist.

5. Verfahren nach Anspruch 1,
bei dem die Vibration in dem Vibrationsschritt eine vertikale Hin- und Herbewegung ist.

6. Verfahren nach Anspruch 2 oder 3,
bei dem die Vibration in dem Vibrationsschritt weiterhin eine vertikale Hin- und Herbewegung enthält.

7. Verfahren nach Anspruch 4,
bei dem die Vibration in dem Vibrationsschritt weiterhin eine vertikale Hin- und Herbewegung enthält.

8. Verfahren nach einem der Ansprüche 1 bis 7,
bei dem der nadelähnliche vertiefte Abschnitt aufweist:
einen in der ersten Oberfläche der Form befindlichen Becherabschnitt; und
einen mit dem Becherabschnitt verbundenen Distalende-Vertiefungsabschnitt, der eine sich verjüngende Form in Tiefenrichtung der Form besitzt,
ein Kantenabschnitt einer Öffnung des nadelähnlichen vertieften Abschnitts abgefast ist, und
eine Abfasung des Kantenabschnitts einem Krümmungsradius von 30 µm oder mehr und 300 µm oder weniger aufweist.

9. Verfahren nach einem der Ansprüche 1 bis 8,
bei dem die Vibration in dem Vibrationsschritt eine Amplitude von 1 mm oder mehr und 10 mm oder weniger aufweist, eine Frequenz von 15 Hz oder mehr und 100 Hz oder weniger aufweist, und eine Zeit von 1 Sekunde oder länger und 180 Sekunden oder weniger dauert.

10. Verfahren nach einem der Ansprüche 1 bis 9, weiterhin umfassend:
im Anschluss an den Saugschritt, einen Arzneimittellösungs-Trocknungsschritt des Trocknens der Arzneimittellösung, die den nadelähnlichen vertieften Abschnitt ausfüllt;
im Anschluss an den Arzneimittellösungs-Trocknungsschritt, einen Basismateriallösungs-Füllschritt des Füllens der Form mit einer Basismateriallösung; und
einen Basismateriallösungs-Trocknungsschritt des Trocknens der eingefüllten Basismateriallösung.

## Revendications

1. Procédé de fabrication d'un réseau de micro-aiguilles, comprenant :
une étape d'ajustement de positionnement et d'ajustement d'un moule présentant une première surface et une seconde surface, lesquelles sont opposées l'une à l'autre, et une pluralité de portions en retrait similaires à des aiguilles dans la première surface, et une buse d'éjection, laquelle éjecte une solution médicamenteuse dans une première direction ;
une étape de déplacement relatif pour déplacer le moule et la buse d'éjection l'un par rapport à l'autre afin de faire coïncider une position de la portion en retrait similaire à une aiguille avec une position de la buse d'éjection l'une avec l'autre dans une vue de dessus dans la première direction ;
une étape d'éjection pour éjecter la solution médicamenteuse de la buse d'éjection vers la portion en retrait similaire à une aiguille ;
une étape de vibration pour faire vibrer le moule et déplacer la solution médicamenteuse vers la portion en retrait similaire à une aiguille, et fermer la portion en retrait similaire à une aiguille avec la solution médicamenteuse après l'étape d'éjection, et
une étape d'aspiration pour aspirer la seconde surface du moule.

2. Procédé de fabrication d'un réseau de micro-aiguilles selon la revendication 1,
dans lequel la vibration lors de l'étape de vibration est un mouvement de va-et-vient horizontal.

3. Procédé de fabrication d'un réseau de micro-aiguilles selon la revendication 2,
dans lequel une direction de déplacement de la solution médicamenteuse et une direction du mouvement de va-et-vient horizontal sont la même direction.

4. Procédé de fabrication d'un réseau de micro-aiguilles selon la revendication 1,
dans lequel la vibration lors de l'étape de vibration est un mouvement circulaire horizontal.

5. Procédé de fabrication d'un réseau de micro-aiguilles selon la revendication 1,
dans lequel la vibration lors de l'étape de vibration est un mouvement de va-et-vient vertical.

6. Procédé de fabrication d'un réseau de micro-aiguilles selon la revendication 2 ou 3,
dans lequel la vibration lors de l'étape de vibration inclut en outre un mouvement de va-et-vient vertical.

7. Procédé de fabrication d'un réseau de micro-aiguilles selon la revendication 4,
dans lequel la vibration lors de l'étape de vibration inclut en outre un mouvement de va-et-vient vertical.

8. Procédé de fabrication d'un réseau de micro-aiguilles selon l'une quelconque des revendications 1 à 7,
dans lequel la portion en retrait similaire à une aiguille comprend :
une portion en forme de coupe prévue dans la première surface du moule, et
une portion en retrait d'extrémité distale, laquelle est reliée à la portion en forme de coupe et présente une forme présentant un amincissement progressif dans une direction de profondeur du moule ;
une portion de bord d'une ouverture de la portion en retrait similaire à une aiguille est chanfreinée, et
un chanfrein de la portion de bord présente un rayon de courbure supérieur ou égal à 30 µm et inférieur ou égal à 300 µm.

9. Procédé de fabrication d'un réseau de micro-aiguilles selon l'une quelconque des revendications 1 à 8,
dans lequel la vibration lors de l'étape de vibration présente une amplitude supérieure ou égale à 1 mm et inférieure ou égale à 10 mm, une fréquence supérieure ou égale à 15 Hz et inférieure ou égale à 100 Hz, et un temps supérieur ou égal à 1 seconde ou inférieur ou égal à 180 secondes.

10. Procédé de fabrication d'un réseau de micro-aiguilles selon l'une quelconque des revendications 1 à 9, comprenant en outre :
après l'étape d'aspiration, une étape de séchage de solution médicamenteuse pour sécher la solution médicamenteuse remplissant la portion en retrait similaire à une aiguille ;
après l'étape de séchage de solution médicamenteuse, une étape de remplissage de solution de matériau de base pour remplir le moule avec la solution de matériau de base, et
une étape de séchage de solution de matériau de base pour sécher la solution de matériau de base remplie.
